# EUROPEAN PATENT APPLICATION

(11) **EP 4 578 955 A1**
(43) Date of publication of application: **02.07.2025**
(21) Application number: 22956173.3
(22) Date of filing: 30.08.2022
(51) Int. Cl.: C12P 19/40, C12N 9/10

(54) **METHOD FOR ENZYME-CATALYZED SYNTHESIS OF PURINE NUCLEOSIDE AND COMPOSITION**

(30) Priority: 24.08.2022 CN 202211021644
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); LI, Mingji, Tianjin 300457 (CN); LI, Rui, Tianjin 300457 (CN); ZHANG, Yanqing, Tianjin 300457 (CN); DING, Shimao, Tianjin 300457 (CN); YANG, Yiming, Tianjin 300457 (CN); LI, Juan, Tianjin 300457 (CN)
(74) Representative: Zacco GmbH
(86) International application number: PCT/CN2022/115944
(87) International publication number: WO 2024/040628

(57) **Abstract**

Provided are a method for synthesizing a purine nucleoside through enzymatic catalysis and a composition. The method includes catalyzing a substrate with a purine nucleoside phosphorylase and any one of the following enzymes: a pyrimidine nucleoside phosphorylase or a thymidine phosphorylase, to synthesize the purine nucleoside; the substrate includes a substrate nucleoside and a substrate base; the pyrimidine nucleoside phosphorylase includes PyNP, and the PyNP is a protein shown as SEQ ID NO: 1; the thymidine phosphorylase includes TP, and the TP is a protein shown as SEQ ID NO: 2; and the purine nucleoside phosphorylase includes PNP, and the PNP is a protein shown as SEQ ID NO: 3. The method can solve the problem of low production of purine nucleosides synthesized by enzymatic methods in the prior art, and is suitable for the field of enzymatic catalysis.

## Description

The present application is based upon and claims priority to a Chinese application No. 202211021644.X, filed on August 24, 2022, the public contents of which are incorporated herein again as a whole by reference.

### Technical Field

The present application relates to the field of enzymatic catalysis and specifically relates to a method for synthesizing a purine nucleoside through enzymatic catalysis and a composition.

### Background

Purine nucleosides and analogs thereof can be widely used as precursors or final products in the field of medicine, such as antiviral drugs, anti-tumor drugs and other drugs. At present, the purine nucleosides and the analogs thereof are mainly synthesized by chemical methods (WO2021186328A1, CN113173961A, CN111592542A, CN111253455A, CN110627729A, CN102127135A, WO2010113937A1, WO2009058800A2, CN101250210A, US2005171126A1, and EP1108724A2), which have relatively complicated synthesis processes requiring strict reaction conditions. Moreover, products synthesized by the chemical methods have complicated purification steps and are difficult to separate from isomers thereof, leading to the problems of a low yield, a high cost, etc. In addition, toxic agents are used in chemical synthesis, which will usually cause great damage to the environment.

In addition, the purine nucleosides can also be produced by microbial fermentation methods (CN112553135A, CN112574934A, CN113151238A, CN113278596A, CN112143751A, CN112126666A, CN113373100A, CN102171346, CN101120090A, CN1831115A, and CN1270631A). At present, the microbial fermentation methods for production of the purine nucleosides are mostly limited to the production of natural nucleosides, such as adenosine, guanosine, inosine, etc., which consume a long time, have low production and still have a certain distance from industrial production.

In recent years, enzymatic synthesis methods for the purine nucleosides, due to the advantages of few steps, a higher yield, high optical purity, environmental friendliness, etc., can avoid the above problems and thus have been increasingly concerned. The enzymatic synthesis methods for the purine nucleosides can be realized through a variety of routes using different enzymes. For example, a patent application EP1457568A1 discloses a biological enzyme catalytic method, which can use a nucleoside deoxyribosyl transferase II and a nucleosidase to carry out a reaction for 32 h with thymidine and guanine as a substrate so as to synthesize 31 mM of 2'-deoxyguanosine. In addition, in the patent application, the above two enzymes are also used in combination with an adenosine deaminase to carry out a reaction by a two-step method for about 30 h so as to synthesize more than 40 mM of 2'-deoxyguanosine. In addition, a pyrimidine nucleoside phosphorylase and a purine nucleoside phosphorylase can also be used to synthesize the purine nucleosides. A patent application CN102770532A discloses a method capable of biologically converting a pyrimidine nucleoside and a purine base by a pyrimidine nucleoside phosphorylase and a purine nucleoside phosphorylase, which can convert uridine or 2'-deoxyuridine and the purine base into 2,6-diaminopurine nucleoside and 2,6-diaminopurine-2'-deoxynucleoside with a conversion rate of greater than 90%, but the production is less than 5 mM. According to a patent application CN1207417A, a pyrimidine nucleoside phosphorylase and a purine nucleoside phosphorylase that are derived from *Bacillus stearothermophilus* JTS859 as well as uracil and thymine decomposition enzyme solutions that are derived from *Arthrobacter* are used to carry out a reaction for 110 h with thymidine and a corresponding purine base as a substrate so as to produce 14.9 mM of 2'-deoxyguanosine with a yield of 75%, or to carry out a reaction for 90 h so as to obtain 17.7 mM of 2'-deoxyadenosine with a yield of 88.5%. However, all the enzymatic synthesis methods disclosed above have the problems of a low conversion rate, low production or a long reaction time, etc.

### SUMMARY

The main purpose of the present application is to provide a method for synthesizing a purine nucleoside through enzymatic catalysis and a composition, so as to solve the problem of low production of purine nucleosides synthesized by enzymatic methods in the prior art.

In order to achieve the above purpose, according to a first aspect of the present application, a method for synthesizing a purine nucleoside through enzymatic catalysis is provided. The method includes catalyzing a substrate with a purine nucleoside phosphorylase and any one of the following enzymes: a pyrimidine nucleoside phosphorylase or a thymidine phosphorylase, to synthesize the purine nucleoside; the substrate includes a substrate nucleoside and a substrate base; the pyrimidine nucleoside phosphorylase includes PyNP or a protein having 80% or more identity and same functions with the PyNP, and the PyNP is a protein shown as SEQ ID NO: 1 or a protein having 80% or more identity and same functions with TP; the thymidine phosphorylase includes TP or a protein having 80% or more identity and same functions with the TP, and the TP is a protein shown as SEQ ID NO: 2; and the purine nucleoside phosphorylase includes PNP or a protein having 80% or more identity and same functions with the PNP, and the PNP is a protein shown as SEQ ID NO: 3.

Further, the substrate nucleoside is a nucleoside shown in a formula I, R₁ is selected from -H or -OH, and R₂ is selected from -H or -CH₃; and preferably, the substrate nucleoside includes thymidine, uridine, or 2'-deoxyuridine.

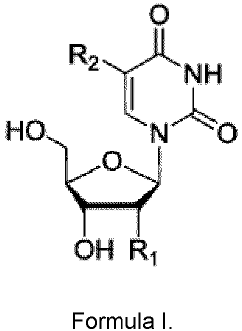

Further, the substrate base is a base shown in a formula II or a formula III, X is selected from -NH₃ or -OCH₃, and Y is selected from -H or -NH₂; and preferably, the substrate base includes guanine, adenine, 2,6-diaminopurine, or 6-methoxyguanine;

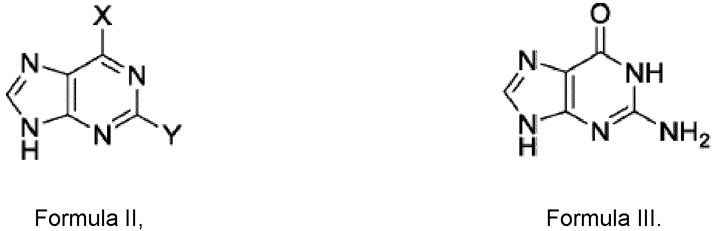

Further, one or more of the PyNP, the TP, or the PNP are a purified protein, a crude enzyme solution, or an immobilized enzyme.

Further, in the method, a concentration of the substrate nucleoside is 2-400 mM, and a concentration of the substrate base is 1-200 mM.

Further, a catalytic time of the enzymatic synthesis is 2-18 h.

Further, a catalytic temperature of the enzymatic synthesis is 60-70°C.

In order to achieve the above purpose, according to a second aspect of the present application, a composition is provided. The composition includes a purine nucleoside phosphorylase and any one of the following enzymes: a pyrimidine nucleoside phosphorylase or a thymidine phosphorylase, wherein the pyrimidine nucleoside phosphorylase is PyNP, and the PyNP is a protein shown as SEQ ID NO: 1; the thymidine phosphorylase is TP, and the TP is a protein shown as SEQ ID NO: 2; and the purine nucleoside phosphorylase is PNP, and the PNP is a protein shown as SEQ ID NO: 3.

Further, one or more of the pyrimidine nucleoside phosphorylase(PyNP), the thymidine phosphorylase (TP), or the purine nucleoside phosphorylase (PNP) are a purified protein, a crude enzyme solution, or an immobilized enzyme; and preferably, the composition includes one or more of the PyNP, the TP, or the PNP as a purified protein, a crude enzyme solution, or an immobilized enzyme.

Further, the composition further includes a substrate nucleoside and a substrate base; the substrate nucleoside is a nucleoside shown in a formula I, R₁ is selected from -H or -OH, and R₂ is selected from -H or -CH₃; the substrate base is a base shown in a formula II or a formula III, X is selected from -NH₃ or -OCH₃, and Y is selected from -H or -NH₂; and preferably, a concentration of the substrate nucleoside is 2-400 mM, and a concentration of the substrate base is 1-200 mM;

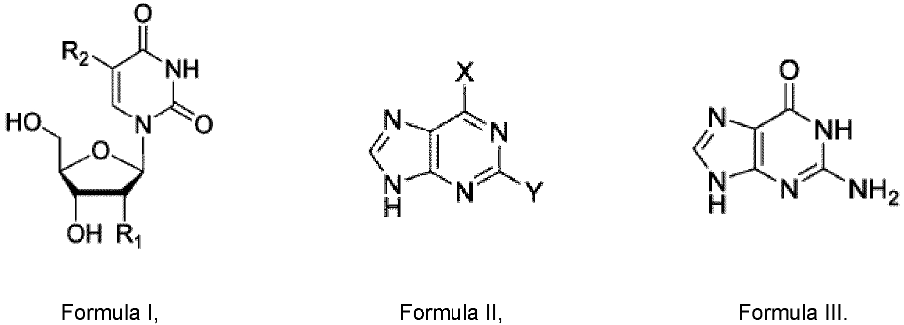

By applying the technical schemes of the present application, the pyrimidine nucleoside phosphorylase and the purine nucleoside phosphorylase, or the thymidine phosphorylase and the purine nucleoside phosphorylase, including the PyNP and the PNP, or the TP and the PNP, are used to perform enzymatic catalysis to catalyze the generation of a plurality of purine nucleosides by a one-step method with the substrate nucleoside and the substrate base as raw materials, and large-scale production can be realized so as to realize efficient synthesis in a short reaction time and increase the production of the purine nucleosides.

### Brief Description of the Drawings

Drawings attached to the specification forming a part of the present application are used to provide further understanding of the present application, and schematic embodiments of the present application and descriptions thereof are used to interpret the present application, rather than to form improper limitations of the present application. In the drawings:
Fig. 1 shows a standard curve diagram of 2'-deoxyadenosine according to Example 5 of the present application.
Fig. 2 shows a standard curve diagram of 2'-deoxyguanosine according to Example 7 of the present application.
Fig. 3 shows a high performance liquid chromatography diagram of the 2'-deoxyadenosine according to Example 5 of the present application.
Fig. 4 shows a high performance liquid chromatography diagram of 2'-deoxyadenosine according to Example 6 of the present application.

### Detailed Description of the Embodiments

It should be noted that the embodiments in the present application and features in the embodiments can be combined with each other without conflict. The present application is described in detail below in conjunction with the embodiments.

As mentioned in the background, all methods for synthesizing purine nucleosides in the prior art have the problems of a high cost, long time consumption, low production, etc., which are not conducive to preparation and subsequent utilization of purine nucleosides and analogs thereof.

Existing enzymatic synthesis methods for nucleoside phosphorylases have many routes and reaction steps, complicated operations, long time consumption, low production, a low yield and a high production cost. For example, according to an enzymatic conversion method involved in a patent application EP1457568A1, reactions in two steps and three enzymes are required, a time of 30 h is consumed, and the production of 2'-deoxyguanosine is only 40 mM. According to an enzymatic conversion method published in CN1207417A, a time of 90 h or above is consumed, the production of a purine nucleoside is low, additional preparation of enzyme solutions with uracil and thymine decomposition activity is required so as to increase the production cost, and the production and the yield are significantly decreased without addition and are only 13% to 50% of that with addition. According to a method published in a patent application CN102770532A, only less than 5 mM of 2,6-diaminopurine nucleoside and 2,6-diaminopurine-2'-deoxynucleoside can be obtained. Because of the problems of low solubility of a purine base substrate, instability of an enzyme and easy destruction of an intermediate product (ribose-1-phosphate or 2'-eoxyribose-1-phosphate), simple amplification of a reaction or extension of a reaction time may destroy a reaction balance and inactivate the enzyme, such that a finally obtained conversion rate and a yield are low, and a production cost is increased. Therefore, the method is difficult to be applied to actual commercial production.

In order to improve the above situations, in the present application, the inventor has tried to explore a new method for synthesizing a purine nucleoside through enzymatic catalysis. It is found from a large number of related proteins with potential activity that PyNP (a pyrimidine nucleoside phosphorylase, a protein shown as SEQ ID NO: 1) and PNP (a purine nucleoside phosphorylase, a protein shown as SEQ ID NO: 3) as well as TP (a thymidine phosphorylase including a protein shown as SEQ ID NO: 2) and PNP have catalytic activity under a combined action, and it is found that enzyme solutions of the PyNP, the TP, and the PNP can be used for catalyzing a substrate nucleoside and a substrate base by a one-step method to generate a target product, namely a purine nucleoside. The enzymatic catalysis method has a short reaction time and high synthesis efficiency, such that the obtained product has high production. On this basis, the applicant puts forward a series of protection schemes of the present application.

In a first typical embodiment of the present application, a method for synthesizing a purine nucleoside through enzymatic catalysis is provided. The method includes catalyzing a substrate with a purine nucleoside phosphorylase and any one of the following enzymes: a pyrimidine nucleoside phosphorylase or a thymidine phosphorylase, to synthesize the purine nucleoside; the substrate includes a substrate nucleoside and a substrate base; the pyrimidine nucleoside phosphorylase includes PyNP, and the PyNP is a protein shown as SEQ ID NO: 1; the thymidine phosphorylase includes TP, and the TP is a protein shown as SEQ ID NO: 2; and the purine nucleoside phosphorylase includes PNP, and the PNP is a protein shown as SEQ ID NO: 3. All the pyrimidine nucleoside phosphorylase, the thymidine phosphorylase, or the purine nucleoside phosphorylase include proteins having 80%, 85%, 90%, 95%, 98%, 99%, 99.5%, or 99.9% identity and same functions with the PyNP, the TP, or the PNP, respectively.

The pyrimidine nucleoside phosphorylase used in the present application is derived from *Thermus thermophilus* and is the protein shown as SEQ ID NO: 1, and the pyrimidine nucleoside phosphorylase is named as the PyNP. The thymidine phosphorylase is derived from *Homo sapiens* and is the protein shown as SEQ ID NO: 2, and the thymidine phosphorylase is named as the TP. The purine nucleoside phosphorylase is derived from *Geobacillus stearothermophilus* and is the protein shown as SEQ ID NO: 3, and the purine nucleoside phosphorylase is named as the PNP. In the enzymatic catalysis method, the two nucleoside phosphatases derived from different sources are used to efficiently synthesize the purine nucleoside by a one-step method, the production cost can be reduced, and the method can be applied to large-scale industrial production of related products. The substrate includes the substrate nucleoside and the substrate base, which can be a natural nucleoside or base structure, or a similar structure modified by humans.

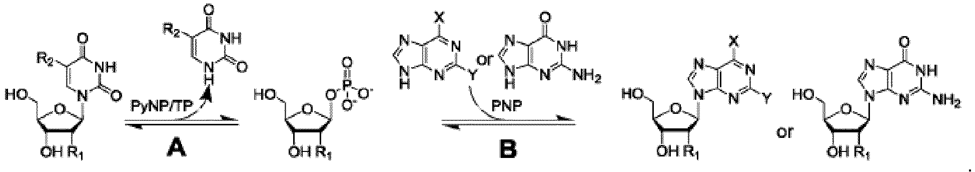

In the enzymatic catalysis method, the above reaction A is carried out under catalysis of the PyNP or the TP, a C-N bond between a pyrimidine group and a ribose group of the substrate nucleoside is broken, and a phosphate group is bound to a nucleoside group to form a ribose phosphate intermediate. Under the action of the PNP, the ribose phosphate intermediate undergoes the above reaction B to form a C-N bond between a C-1' atom of the ribose group and an N-9 atom of the purine substrate base so as to generate a purine nucleoside compound. In the reactions A and B, no additional coenzymes, such as NADPH, are required. The reactions can be completed by only using the substrate and the proteins, and the reactions are simple and are not required to be carried out in living cells, such that the reaction cost and the efficiency are greatly reduced. The enzymatic catalysis method using combination of the 2 proteins can complete a catalytic reaction in a short catalytic time, has a high substrate conversion rate, is also applicable in an amplification reaction, and can realize large-scale industrial production.

In a preferred embodiment, the substrate nucleoside is a nucleoside shown in a formula I, R₁ is selected from -H or -OH, and R₂ is selected from -H or -CH₃; and preferably, the substrate nucleoside includes thymidine, uridine, or 2'-deoxydridine;

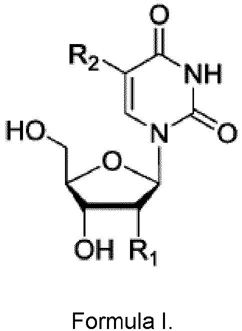

In a preferred embodiment, the substrate base is a base shown in a formula II or a formula III, X is selected from -NH₃ or -OCH₃, and Y is selected from -H or -NH₂; and preferably, the substrate base includes guanine, adenine, 2,6-diaminopurine, or 6-methoxyguanine;

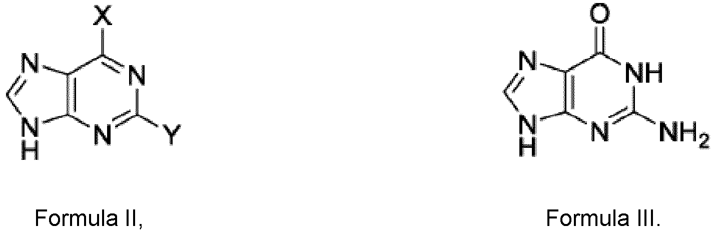

The substrate nucleoside and the substrate base can be used to prepare purine nucleosides, such as 2'-deoxyadenosine, 2'-deoxyguanosine, 2,6-diaminopurine nucleoside, 2,6-diaminopurine-2'-deoxynucleoside and 6-O-methyl guanosine, by the enzymatic catalysis method.

The substituents, including the R₁, the R₂, the X and the **Y,** in the substrate nucleoside and the substrate base are far away from a reaction site. Therefore, different substrates can undergo an enzymatic catalysis reaction with the PyNP and the PNP to prepare a variety of purine nucleosides.

In a preferred embodiment, one or more of the PyNP, the TP, or the PNP are a purified protein, a crude enzyme solution, or an immobilized enzyme.

With the PyNP, the TP and the PNP to catalyze a reaction, the above 3 proteins can exist in various forms, such as a purified protein, a crude enzyme solution, or an immobilized enzyme, and can catalyze the synthesis of the purine nucleoside. A gene expressing the PyNP and/or the TP and/or the PNP is cloned into a host cell, and after protein expression is induced, the host cell can be broken to obtain a crude enzyme solution containing a target protein. The crude enzyme solution is simple to prepare, has good catalytic ability, and can reduce the production cost of a catalytic reaction.

In a preferred embodiment, in the method, a concentration of the substrate nucleoside is 2-400 mM, and a concentration of the substrate base is 1-200 mM.

The enzymatic catalysis method can be used to carry out an amplification reaction. In the reaction system, the maximum concentration of the substrate nucleoside is 400 mM, and the maximum concentration of the substrate base is 200 mM, such that the purine nucleoside is prepared on a large scale.

In a preferred embodiment, a catalytic time of the enzymatic synthesis is 2-18 h.

In a preferred embodiment, a catalytic temperature of the enzymatic synthesis is 60-70°C.

In the suitable catalytic temperature and catalytic time, the enzymatic catalysis reaction can be completed, and the conversion rate of the substrate base, namely the reaction yield, is high. Enzymes or other reagents are not required to be added in the middle of the reaction, the reaction can be completed under catalysis by a one-step method, and the method is suitable for large-scale industrial production.

In a second typical embodiment of the present application, a composition is provided. The composition includes a purine nucleoside phosphorylase and any one of the following enzymes: a pyrimidine nucleoside phosphorylase or a thymidine phosphorylase, wherein, the pyrimidine nucleoside phosphorylase is PyNP, and the PyNP is a protein shown as SEQ ID NO: 1; the thymidine phosphorylase is TP, and the TP is a protein shown as SEQ ID NO: 2; and the purine nucleoside phosphorylase is PNP, and the PNP is a protein shown as SEQ ID NO: 3. The composition is used for synthesizing a purine nucleoside.

In a preferred embodiment, the composition includes a purified protein, a crude enzyme solution, or an immobilized enzyme.

In a preferred embodiment, the composition further includes a substrate nucleoside and a substrate base that are reacted to generate a purine nucleoside; the substrate nucleoside is a nucleoside shown in a formula I, R₁ is selected from -H or -OH, and R₂ is selected from -H or -CH₃; the substrate base is a base shown in a formula II or a formula III, X is selected from -NH₃ or -OCH₃, and Y is selected from -H or -NH₂; and preferably, a concentration of the substrate nucleoside is 2-400 mM, and a concentration of the substrate base is 1-200 mM;

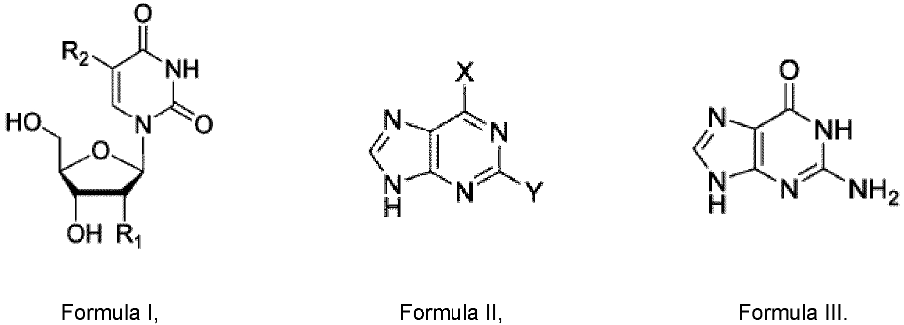

The PyNP is a pyrimidine nucleoside phosphorylase derived from *Thermus thermophilus* and has an amino acid sequence shown as SEQ ID NO: 1. The TP is a thymidine phosphorylase derived from *Homo sapiens* and has an amino acid sequence shown as SEQ ID NO: 2. The PNP is a purine nucleoside phosphorylase derived from *Geobacillus stearothermophilus* and has an amino acid sequence shown as SEQ ID NO: 3. The composition includes the PyNP and the PNP, or the TP and the PNP. The composition can be used for catalyzing a reaction of a nucleoside, such as thymidine or uridine, and a base, such as guanine or adenine, to generate a purine nucleoside and analogs thereof. The proteins in the composition can be independently selected from a purified protein, a crude enzyme liquid, or an immobilized enzyme and other forms, respectively, which can achieve a catalytic effect.

Beneficial effects of the present application are further explained in detail below in combination with specific embodiments.

### Example 1

### 1. Construction of strains

A pyrimidine nucleoside phosphorylase used in the present application was derived from *Thermus thermophilus* and named as PyNP with an amino acid sequence shown as SEQ ID NO: 1. A thymidine phosphorylase was derived from *Homo sapiens* and named as TP with an amino acid sequence shown as SEQ ID NO: 2. A purine nucleoside phosphorylase was derived from *Geobacillus stearothermophilus* and named as PNP with an amino acid sequence shown as SEQ ID NO: 3.

DNA sequences encoding two enzymes were obtained after codon optimization, where a DNA sequence encoding the PyNP was SEQ ID NO: 4; a DNA sequence encoding the TP was SEQ ID NO: 5; and a DNA sequence encoding the PNP was SEQ ID NO: 6. The sequences were cloned into an expression vector pET28a(+), respectively. Plasmids obtained were transferred to competent cells of an *Escherichia coli* BL21(DE3) host to obtain monoclonal strains.
SEQ ID NO: 1: 
MNPVAFIREKREGKKHRREDLEAFLLGYLRDEVPDYQVSAWLMAAFLRGLDPEETLWLTETM
ARSGKVLDLSGLPHPVDKHSSGGVGDKVSLVVGPILAASGCTFAKMSGRGLAHTGGTIDKLESVP
GWRGEMTEAEFLERARRVGLVIAAQSPDLAPLDGKLYALRDVTATVESVPLIASSIMSKKLAAGARS
IVLDVKVGRGAFMKTLEEARLLAKTMVAIGQGAGRRVRALLTSMEAPLGRAVGNAIEVREAIEALKG
EGPGDLLEVALALAEEALRLEGLDPALARKALEGGAALEKFRAFLEAQGGDPRAVEDFSLLPLAEE
HPLRAEREGVVREVDAYKVGLAVLALGGGRKRKGEPIDHGVGVYLLKKPGDRVERGEALALVYHR
RRGLEEALGHLREAYALGEEAHPAPLVLEAI.
SEQ ID NO: 2:  






.
SEQ ID NO: 3: 
MSVHIGAKEHEIADKILLPGDPLRAKYIAETFLEGATCYNQVRGMLGFTGTYKGHRISVQGTG
MGVPSISIYITELMQSYNVQTLIRVGTCGAIQKDVKVRDVILAMTSSTDSQMNRMTFGGIDYAPTAN
FDLLKTAYEIGKEKGLQLKVGSVFTADMFYNENAQFEKLARYGVLAVEMETTALYTLAAKFGRKALS
VLTVSDHILTGEETTAEERQTTFNEMIEVALETAIRQ.
SEQ ID NO: 4: 
atgaacccggtggcgtttattcgcgaaaaacgcgaaggcaagaaacatcgccgcgaggatctggaagcgtttctgctgggctatct
gcgcgatgaagtgccggattatcaggtgagcgcgtggctgatggcggcgtttttacgcggcttagatccggaagaaaccctgtggctgac
cgaaacgatggcccgcagcggtaaagtgctggatctgagcggtctgccgcatccggttgataaacatagcagcggcggcgtgggcgat
aaagtgagcttagtggtgggcccgattctggcggcgagcggttgtacctttgcgaaaatgtcgggccgcggcctggcgcataccggtggt
accattgataaactggaaagcgtgccgggctggcgcggcgaaatgaccgaagcagaatttctggaacgcgcccgtcgcgttggtctggtt
attgcggcgcaaagcccggatttagcgccgttagatggcaaactgtatgcgctgcgcgatgtgaccgcgaccgttgaatcagttccgctga
ttgcgagcagcattatgagcaagaaactggcggcgggcgcccgtagcattgtgttagatgttaaagtgggccgcggcgcgtttatgaaaa
ccctggaagaagcgcgtctgctggcgaaaacgatggtggcgattggccagggcgcgggccgtcgtgttcgtgcgttattaaccagcatgg
aagcgcctctgggtcgtgcggttggcaatgcgattgaagtgcgtgaagcgattgaagcgctgaaaggcgaaggcccgggcgatttatta
gaagtggcgctggcgttagcggaagaagcgctgcgtttagaaggcttagatccggcgctggcgcgtaaagcgttagaaggtggcgcgg
cgctggaaaaatttcgcgcgtttctggaagcgcagggcggcgatccgcgtgcagttgaagattttagcctgctgccgctggcggaagaac
atccgttacgtgcggaacgcgaaggcgtggttcgcgaagttgatgcgtataaagtgggcctggcggtgctggcgttaggcggtggtcgta
aacgtaaaggtgaaccgattgatcatggcgtgggcgtgtatctgctgaagaaaccgggcgatcgcgtggaacgcggcgaagcgttagc
gttagtgtatcatcgccgccgcggcttagaagaagcgctgggtcatttacgcgaagcgtatgcgctgggcgaagaagcgcatccggcgc
ctctggttttagaagcgatttaa.
SEQ ID NO: 5: 
atggcggcgttaatgacccctggcaccggtgcgcctcctgcgcctggtgattttagcggcgaaggcagccagggcctgccggacc
ctagccctgaacctaaacaactgccggaactgattcgcatgaaacgcgatggtggccgcctgagcgaagcggatattcgcggttttgtgg
cggcggtggtgaacggcagcgcacaaggtgcgcaaattggtgcgatgttaatggcgattcgcctgcgcggcatggatctggaagaaac
 
cagcgtgctgacccaggcgctggcgcaaagcggtcagcaactggaatggcctgaagcgtggcgccagcagttagttgataaacatagc
accggcggcgtgggcgataaagtgagcttagttctggcgccggcgctggcggcgtgtggctgtaaagttcctatgattagcggccgtggcc
tgggccataccggtggtactttagataaactggaaagcattccgggctttaacgtgattcagagcccggaacagatgcaggtgctgctgga
tcaggcgggctgttgcattgtgggccagagcgaacagctggtgccggcggaagggattctgtatgcggcgcgtgatgtgaccgcgaccg
ttgatagcttaccgctgattaccgcgagcattctgagcaaaaagctggtggaaggcctgagcgccctggtggtggatgttaaatttggcggc
gcggcggtgtttccgaaccaggaacaagcccgcgaactggcgaaaaccctggtgggcgttggcgcgagcctgggtttacgtgttgcggc
ggcgttaaccgcgatggataaaccgttaggccgctgcgttggccatgcgctggaagtggaagaagcgctgctgtgcatggatggcgcgg
gccctcctgatttacgcgatctggttacgaccctgggcggcgcgttactgtggttaagcggccatgcgggtacccaggcgcaaggcgcag
cgcgtgttgcggcagcgctggatgatggtagcgcgttaggtcgttttgaacgcatgctggcggcgcagggtgtggaccctggtttggcgcgt
gcgttatgtagcggcagccctgcggaacgtcgccagttattacctcgcgcccgtgaacaagaagaactgctggcgccggcggatggcac
cgttgaattagtgcgtgcgctgccgttagcgctggtgttacatgaactgggcgcgggccgctcacgtgcgggtgaacctttacgtttaggcgtt
ggtgcggaactgctggtggatgttggtcagcgcctgcgccgtggtaccccttggttacgtgttcatcgtgatggtccggcgctgagcggccct
caaagccgtgcgctgcaagaagcgttagttctgagcgatcgtgcgccgtttgcggcgccgagcccttttgcggaattggttttaccgcctca
gcaataa.
SEQ ID NO: 6:

atgagcgtgcatattggcgcgaaagaacatgaaattgcggataaaattctgctgccgggcgatccgttacgcgcgaaatatattgc
ggaaacctttctggaaggcgcgacctgctataaccaggtgcgcggcatgctgggctttaccggcacctataaaggccatcgcattagcgt
gcagggcaccggcatgggcgttcctagcatttcaatttatattaccgaactgatgcagagctataacgtgcagaccctgattcgcgtgggca
cctgcggtgcgattcaaaaagatgtgaaagtgcgcgatgtgattctggcgatgaccagcagcaccgatagccagatgaaccgcatgacc
tttggcggcattgattatgcgccgaccgcgaactttgatctgctgaaaaccgcgtatgaaattggcaaagaaaaaggcctgcagctgaaa
gtgggcagcgtgtttaccgcggatatgttttataacgaaaacgcgcagtttgagaaactggcccgctatggcgtgctggcggttgaaatgg
aaaccaccgcgctgtataccctggcggcgaaatttggccgcaaagcgctgagcgtgctgaccgtgagcgatcatattctgaccggcgaa
gaaaccaccgcggaagaacgccagaccacctttaacgaaatgattgaagtggcgctggaaaccgcgattcgccagtaa.

### 2. Protein expression

The *Escherichia coli* strains expressing the PyNP, the TP, and the PNP were inoculated into a test tube, respectively, cultured at 37°C for 16 h, then inoculated into a 2 L shaker containing 500 mL of an LB liquid culture medium at an inoculation amount of 1 v/v%, and cultured at 37°C until an OD₆₀₀ value was 0.6. Isopropyl-β-D-thiogalactoside with a final concentration of 0.1 M was added to induce protein expression, and culturing was performed at 20°C for 18 h. After the culturing was completed, bacterial solutions were subjected to centrifugation at 7,000 rpm for 10 min, and bacteria were collected for later use.

### 3. Preparation of enzyme solutions

0.1 g of bacterial sludge was weighed, 1 mL of a potassium phosphate buffer solution with a pH value of 7.5 was added and evenly mixed by shaking, and a bacterial suspension was broken by an ultrasonic crusher at a powder of 30% for 5 min.

### 4. HPLC detection method

A chromatographic column was Atlantis T3 Column, 4.6 mm x 150 mm, a mobile phase was methanol containing 0.1 v/v% of trifluoroacetic acid (TFA), a flow rate was 1 mL/min, a column temperature was 40°C, a UV detector was used, a detection wavelength was 254 nm, and a detection time was 15 min.

### Example 2 Enzymatic catalysis of purine nucleosides and analogs thereof

1 mL of a reaction system was prepared with a 2 mM phosphate buffer solution (pH 7.5), including 2 mM thymidine or uridine, 1 mM purine or purine analogs, a PyNP enzyme solution prepared from different weights of bacterial sludge and a PNP enzyme solution prepared from different weights of bacterial sludge, and then a reaction was carried out at 60°C for 18 h. After the reaction was completed, 1 mL of DMSO was added and then sent for HPLC detection. Experimental results are shown in Table 1. The results show that reaction conversion rates of adenine and purine analogs are all higher than 79%, and in the synthesis of 2,6-diaminopurine nucleoside and 2,6-diaminopurine -2'-deoxynucleoside, the conversion rate of 2,6-diaminopurine can reach 96%.

**Table 1. Enzymatic catalysis of purine nucleosides and analogs thereof**

| PyNP enzyme amount (mg) | PNP enzyme amount (mg) | Substrate | Bases and analogs thereof | Product | Conversion rate of purine (%) |
|---|---|---|---|---|---|
| 0.48 | 0.27 | Thymidine | Adenine | 2'-deoxyadenosine | 81.37 |
| 2.4 | 1.5 | Thymidine | Guanine | 2'-deoxyguanosine | 79.47 |
| 0.12 | 0.08 | Uridine | 2,6-diaminopurine | 2,6-diaminopurine nucleoside | 96.30 |
| 0.24 | 1.5 | Thymidine | 2,6-diaminopurine | 2,6-diaminopurine-2'-deoxynucleoside | 96.04 |

### Example 3 Effect of increase of a substrate concentration on enzymatic catalysis of purine nucleosides and analogs thereof

1 mL of a reaction system was prepared with a 2 mM phosphate buffer solution (pH 7.5), including 4 mM thymidine or uridine, 1 mM purine or purine analogs, a PyNP enzyme solution prepared from different weights of bacterial sludge and a PNP enzyme solution prepared from different weights of bacterial sludge, and then a reaction was carried out at 70°C for 2 h. After the reaction was completed, 1 mL of DMSO was added and then sent for HPLC detection. Experimental results are shown in Table 2. The results show that when the thymidine substrate concentration is increased and the time is shortened to 2 h, reaction conversion rates of adenine and purine analogs thereof are still increased and are all higher than 90%.

**Table 2. Effect of enzyme amounts on enzymatic catalysis of purine nucleosides and analogs thereof**

| PyNP enzyme amount (mg) | PNP enzyme amount (mg) | Substrate | Bases and analogs thereof | Product | Conversion rate of purine (%) |
|---|---|---|---|---|---|
| 0.96 | 0.27 | Thymidine | Adenine | 2'-deoxyadenosine | 90.35 |
| 4.8 | 1.5 | Thymidine | Guanine | 2'-deoxyguanosine | 96.54 |
| 0.24 | 0.08 | Uridine | 2,6-diaminopurine | 2,6-diaminopurine nucleoside | 99.18 |
| 0.48 | 1.5 | Thymidine | 2,6-diaminopurine | 2,6-diaminopurine-2'-deoxynucleoside | 98.99 |

### Example 4 Optimization of enzyme amounts in a synthetic reaction of 2'-deoxyadenosine

1 mL of a reaction system was prepared with a 2 mM phosphate buffer solution (pH 7.5), including 400 mM thymidine, 200 mM adenine, a PyNP enzyme solution prepared from 9.7-48.4 mg of bacterial sludge and a PNP enzyme solution prepared from 2.7-13.5 mg of bacterial sludge, and then a reaction was carried out at 60°C for 16 h. After the reaction was completed, 1 mL of DMSO was added and then sent for HPLC detection. Experimental results are shown in Table 3. The results show that when lower amounts of enzymes are used, the conversion rate of purine can still be maintained at 90% or above. For example, when the PyNP enzyme solution prepared from 9.7 mg of the bacterial sludge and the PNP enzyme solution prepared from 2.7 mg of the bacterial sludge are used, the conversion rate is 93.17%.

**Table 3. Optimization of enzyme amounts in a synthetic reaction of 2'-deoxyadenosine**

| PyNP bacterial sludge (mg) | PNP bacterial sludge (mg) | Conversion rate of thymidine (%) | Conversion rate of purine (%) |
|---|---|---|---|
| 9.7 | 13.5 | 43.58 | 91.06 |
| 19.4 | 13.5 | 42.75 | 92.58 |
| 29.0 | 13.5 | 43.16 | 91.62 |
| 38.7 | 13.5 | 43.67 | 93.18 |
| 48.4 | 13.5 | 49.62 | 93.98 |
| 48.4 | 10.8 | 46.68 | 93.68 |
| 48.4 | 8.1 | 45.26 | 93.48 |
| 48.4 | 5.4 | 44.82 | 93.69 |
| 48.4 | 2.7 | 46.76 | 92.45 |
| 9.7 | 2.7 | 44.32 | 93.17 |
| 19.4 | 5.4 | 46.88 | 92.60 |
| 29.0 | 8.1 | 44.50 | 93.25 |
| 38.7 | 10.8 | 45.34 | 92.98 |

### Example 5 Amplification reaction for synthesis of 2'-deoxyadenosine

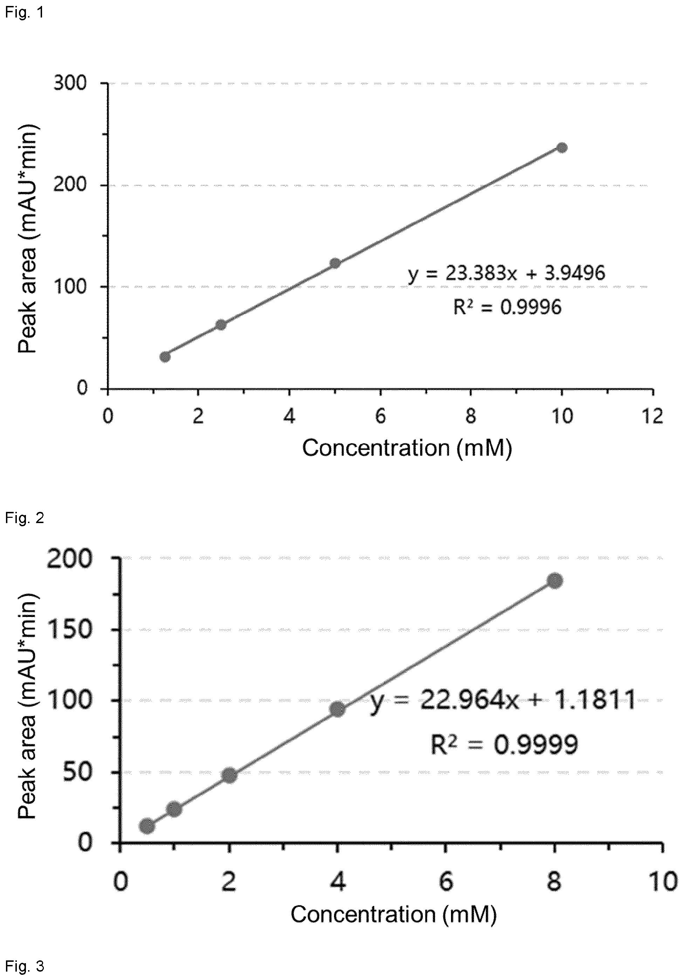

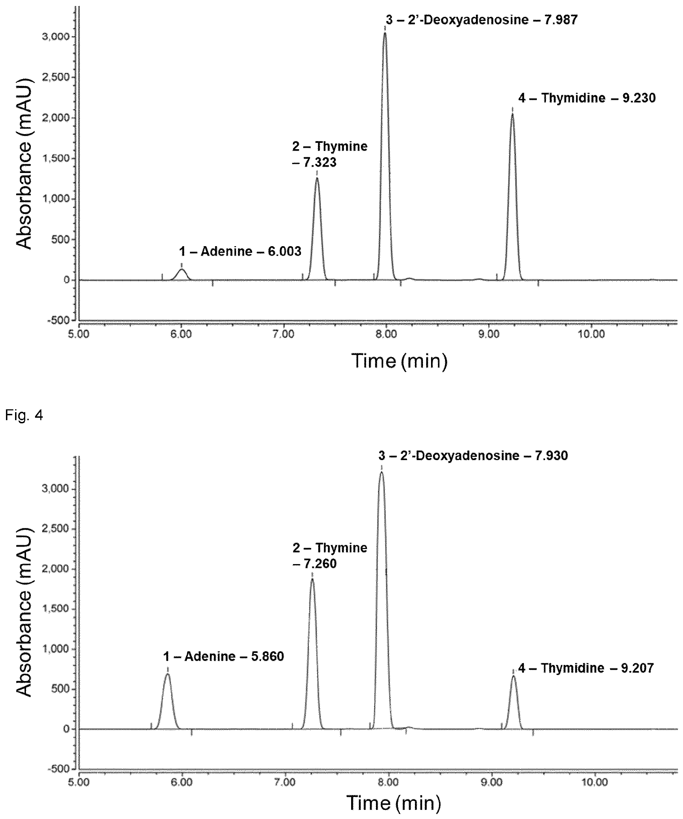

50 mL of a reaction system was prepared with a 2 mM phosphate buffer solution (pH 7.5), including 400 mM thymidine, 200 mM adenine, a PyNP enzyme solution prepared from 484.0 mg of bacterial sludge and a PNP enzyme solution prepared from 135.0 mg of bacterial sludge, and then a reaction was carried out at 60°C for 16 h to generate thymine and 2'-deoxyadenosine. After the reaction was completed, 1 mL of a reaction solution was taken, added to an equal volume of DMSO for dissolution and then sent for HPLC detection after being diluted by 20 times. As shown in Fig. 3, the conversion rate of thymidine is 39.20%, and the conversion rate of purine is 94.72%. According to a standard curve shown in Fig. 1, the production of 2'-deoxyadenosine is calculated as 51.1 g/L.

### Example 6 Effect of a supplemented enzyme amount on the amplification reaction for synthesis of 2'-deoxyadenosine

After Example 5 was implemented, an enzyme solution prepared from 1.35 g of adenine and 135.0 mg of PNP bacterial sludge was supplemented to carry out the reaction continuously for 24 h. After the reaction was completed, 1 mL of a reaction solution was taken, added to an equal volume of DMSO for dissolution and then sent for HPLC detection after being diluted by 20 times. As shown in Fig. 4, the conversion rate of thymidine is 74.97%, and the conversion rate of purine is 81.30%. According to the standard curve shown in Fig. 1, the production of 2'-deoxyadenosine is calculated as 68.3 g/L.

### Example 7 Enzymatic catalysis reaction of 2'-deoxyguanosine

1. Guanine with a concentration of 1 M was prepared and dissolved in NaOH; 120 mM thymidine, 40 mM guanine, a PyNP enzyme solution prepared from 58 mg of bacterial sludge and a PNP enzyme solution prepared from 120 mg of bacterial sludge were added to 1 mL of a reaction system of a 2 mM phosphate buffer solution (pH 7.5); and after a reaction was carried out at 60°C for 16 h, a sample was taken, added to an equal volume of DMSO for dissolution and then sent for HPLC detection after being diluted by 20 times. The conversion rate of thymidine is 37.77%, and the conversion rate of purine is 86.42%. According to a standard curve shown in Fig. 2, the maximum production of 2'-deoxyguanosine is calculated as 9.8 g/L.
2. Guanine with a concentration of 1 M was prepared and dissolved in NaOH; 240 mM thymidine, 80 mM guanine, a PyNP enzyme solution prepared from 58 mg of bacterial sludge and a PNP enzyme solution prepared from 120 mg of bacterial sludge were added to 1 mL of a reaction system of a 2 mM phosphate buffer solution (pH 7.5); and after a reaction was carried out at 60°C for 16 h, a sample was taken, added to an equal volume of DMSO for dissolution and then sent for HPLC detection after being diluted by 20 times. The conversion rate of thymidine is 31.79%, and the conversion rate of purine is 77.07%. According to the standard curve shown in Fig. 2, the maximum production of 2'-deoxyguanosine is calculated as 17.5 g/L.

Experimental results are shown in Table 4.

**Table 4. Catalysis reaction of 2'-deoxyguanosine**

| Thymidine (mM) | Guanine (mM) | PyNP bacterial sludge (mg) | PNP bacterial sludge (mg) | Conversion rate of thymidine (%) | Conversion rate of purine (%) | Production (g/L) |
|---|---|---|---|---|---|---|
| 120 | 40 | 58 | 120 | 37.77 | 86.42 | 9.8 |
| 240 | 80 | 58 | 120 | 31.79 | 77.07 | 17.5 |

### Example 8 Reaction for synthesis of 2,6-diaminopurine nucleoside

10 mL of a reaction system was prepared with a 2 mM phosphate buffer solution (pH 7.5), including 320 mM uridine, 200 mM 2,6-diaminopurine, a PyNP enzyme solution prepared from 195.4 mg of bacterial sludge and a PNP enzyme solution prepared from 150.1 mg of bacterial sludge, and then a reaction was carried out at 60°C for 16 h. After the reaction was completed, 1 mL of a reaction solution was taken, added to an equal volume of DMSO for dissolution and then sent for HPLC detection after being diluted by 20 times. The conversion rate of uridine is 54.92%, the conversion rate of purine is 96.18%, and the production of 2,6-diaminopurine nucleoside is 54.3 g/L.

### Example 9 Reaction for synthesis of 2,6-diaminopurine-2'-deoxynucleoside

10 mL of a reaction system was prepared with a 2 mM phosphate buffer solution (pH 7.5), including 400 mM 2'-deoxyuridine, 200 mM 2,6-diaminopurine, a TP enzyme solution prepared from 230.0 mg of bacterial sludge and a PNP enzyme solution prepared from 75.0 mg of bacterial sludge, and then a reaction was carried out at 50°C for 16 h. After the reaction was completed, 1 mL of a reaction solution was taken, added to an equal volume of DMSO for dissolution and then sent for HPLC detection after being diluted by 20 times. The conversion rate of 2'-deoxyuridine is 43.89%, the conversion rate of purine is 97.75%, and the production of 2,6-diaminopurine-2'-deoxynucleoside is 59.6 g/L.

### Example 10 Enzymatic catalysis reaction for synthesis of 6-O-methyl guanosine

10 mL of a reaction system was prepared with a 2 mM phosphate buffer solution (pH 7.5), including 320 mM uridine, 200 mM 6-O-methylguanine, a PyNP enzyme solution prepared from 195.4 mg of bacterial sludge and a PNP enzyme solution prepared from 165.0 mg of bacterial sludge, and then a reaction was carried out at 60°C for 24 h. After the reaction was completed, 1 mL of a reaction solution was taken, added to an equal volume of DMSO for dissolution and then sent for HPLC detection after being diluted by 20 times. The conversion rate of uridine is 51.27%, the conversion rate of purine is 98.67%, and the production of 6-O-methyl guanosine is 58.6 g/L.

From the above description, it can be seen that the above embodiments of the present application achieve the following technical effects: with the above enzymatic catalysis method, purine nucleoside products can be synthesized with the substrate base and the substrate nucleoside as a substrate in a short catalytic time, and large-scale production can be realized to efficiently synthesize the 2'-deoxyadenosine, the 2'-deoxyguanosine, the 2,6-diaminopurine nucleoside, the 2,6-diaminopurine-2'-deoxynucleoside and the 6-O-methyl guanosine in a short reaction time, where the production can reach 68.3 g/L, 17.5 g/L, 54.3 g/L, 59.6 g/L and 58.6 g/L, respectively.

The statements above are merely preferred embodiments of the present application and are not intended to limit the present application, and for persons skilled in the art, various modifications and alterations of the present application can be made. Any modifications, equivalent substitutions, improvements and the like that are made within the spirit and principles of the present application shall be included in the scope of protection of the present application.

## Claims

1. A method for synthesizing a purine nucleoside through enzymatic catalysis, comprising catalyzing a substrate with a purine nucleoside phosphorylase and any one of the following enzymes: a pyrimidine nucleoside phosphorylase or a thymidine phosphorylase, to synthesize the purine nucleoside, wherein
the substrate comprises a substrate nucleoside and a substrate base;
the pyrimidine nucleoside phosphorylase comprises PyNP, and the PyNP is a protein shown as SEQ ID NO: 1;
the thymidine phosphorylase comprises TP, and the TP is a protein shown as SEQ ID NO: 2; and
the purine nucleoside phosphorylase comprises PNP, and the PNP is a protein shown as SEQ ID NO: 3.

2. The method according to claim 1, wherein the substrate nucleoside is a nucleoside shown in a formula I, wherein R₁ is selected from -H or -OH, R₂ is selected from -H or -CH₃, and 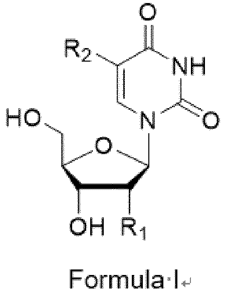 ; and
preferably, the substrate nucleoside comprises thymidine, uridine, or 2'-deoxyuridine.

3. The method according to claim 1, wherein the substrate base is a base shown in a formula II or a formula III, wherein X is selected from -NH₃ or -OCH₃, Y is selected from -H or -NH₂, 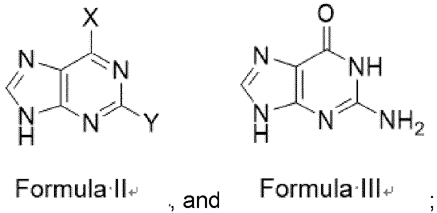 and
preferably, the substrate base comprises guanine, adenine, 2,6-diaminopurine, or 6-methoxyguanine.

4. The method according to claim 1, wherein one or more of the PyNP, the TP, or the PNP are a purified protein, a crude enzyme solution, or an immobilized enzyme.

5. The method according to claim 1, wherein in the method, a concentration of the substrate nucleoside is 2-400 mM, and a concentration of the substrate base is 1-200 mM.

6. The method according to claim 1, wherein a catalytic time of the enzymatic synthesis is 2-18h.

7. The method according to claim 1, wherein a catalytic temperature of the enzymatic synthesis is 60-70°C.

8. A combination, wherein comprising a purine nucleoside phosphorylase and any one of the following enzymes: a pyrimidine nucleoside phosphorylase or a thymidine phosphorylase,
the pyrimidine nucleoside phosphorylase is PyNP, and the PyNP is a protein shown as SEQ ID NO: 1;
the thymidine phosphorylase is TP, and the TP is a protein shown as SEQ ID NO: 2; and
the purine nucleoside phosphorylase is PNP, and the PNP is a protein shown as SEQ ID NO: 3.

9. The combination according to claim 8, wherein one or more of the PyNP, the TP, or the PNP are a purified protein, a crude enzyme solution, or an immobilized enzyme.

10. The combination according to claim 8 or 9, the combination further comprises a substrate nucleoside and a substrate base, wherein
the substrate nucleoside is a nucleoside shown in a formula I, wherein R₁ is selected from -H or -OH, R₂ is selected from -H or -CH₃;
the substrate base is a base shown in a formula II or a formula III, wherein X is selected from -NH₃ or -OCH₃, and Y is selected from -H or -NH₂; 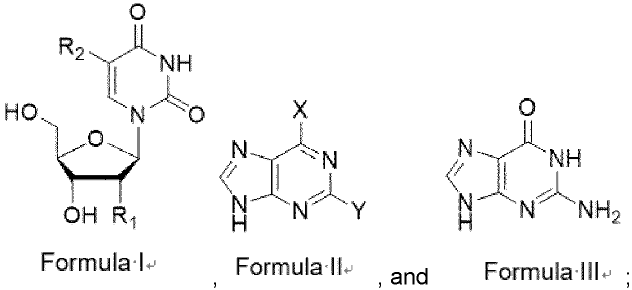 and
preferably, a concentration of the substrate nucleoside is 2-400 mM, and a concentration of the substrate base is 1-200 mM.
